# EUROPÄISCHE PATENTANMELDUNG

(11) **EP 1 371 626 A2**
(43) Veröffentlichungstag der Anmeldung: **17.12.2003**
(21) Anmeldenummer: 03012500.9
(22) Anmeldetag: 02.06.2003
(51) Int. Cl.: C07C 51/31, C07C 51/60, C07C 65/21

(54) **Verfahren zur Herstellung von 3-Alkoxy-2-methylbenzoesäuren**

(30) Priorität: 13.06.2002 DE 10226218
(71) Anmelder: Bayer Aktiengesellschaft, 51368 Leverkusen (DE)
(72) Erfinder: Scherer, Johannes, Dr., 51375 Leverkusen (DE); Mueller-Hauck, Friedrich, Dr., 25557 Bendorf (DE)

(57) **Zusammenfassung**

Die Erfindung betrifft ein verbessertes Verfahren zur Herstellung von 3-Alkoxy-2-methylbenzoesäuren durch Erhitzen von substituierten Naphthalinen in Gegenwart von Alkalimetallhydroxiden und anschließender Alkylierung.

## Beschreibung

Die Erfindung betrifft ein verbessertes Verfahren zur Herstellung von 3-Alkoxy-2-methylbenzoesäuren durch Erhitzen von substituierten Naphthalinen in Gegenwart von Alkalimetallhydroxiden und anschließender Alkylierung.

3-Alkoxy-2-methylbenzoesäuren, wie zum Beispiel 3-Methoxy-2-methylbenzoesäure sind wertvolle Zwischenprodukte bei der Herstellung von Arzneimitteln und Agrochemikalien wie zum Beispiel Insektiziden (siehe z.B. US-A 5,484,926 und EP-A 639 559).

Gemäß Dean et al. (J. Chem. Soc., 1961, 2773) kann man 3-Methoxy-2-methylbenzoesäure herstellen, indem man Natrium-hydrogen-3-amino-naphthalin-1,5-disulfonsäure mit zwei Gewichtsäquivalenten Natriumhydroxid und Wasser bei einem Stickstoffdruck von 40 bar auf 275 bis 280°C erhitzt und nach Abkühlen des Reaktionsgemisches zunächst die 3-Hydroxy-2-methylbenzoesäure durch Filtration und Ansäuern gewinnt und anschließend in nicht näher umschriebener Weise mit Dimethylsulfat umsetzt.

Die Methode besitzt jedoch den Nachteil, dass große Mengen von Alkalien eingesetzt werden müssen, die bei der Aufarbeitung für eine große Menge an Abfallsalzen sorgen. Weiterhin muss die intermediär anfallende 3-Hydroxy-2-methylbenzoesäure zunächst isoliert werden, bevor die weitere Umsetzung erfolgt.

Es bestand daher das Bedürfnis, ein effizientes Verfahren zu entwickeln, das die Herstellung von 3-Alkoxy-2-methylbenzoesäure in vorteilhafter Weise ermöglicht.

Es wurde nun ein Verfahren zur Herstellung von Verbindungen der Formel (I) gerunden, 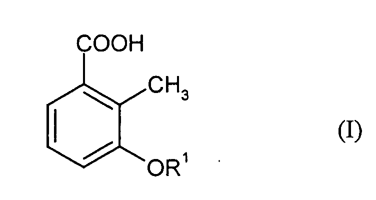 in der
- R¹: für C₁-C₁₄-Alkyl, C₇-C₂ₒ-Arylalkyl, C₁₃-C₂₀-Diarylalkyl oder Reste der Formeln (IIa) oder (IIb) steht

A-OR² (IIa)

A-NR³R⁴ (IIb)
in der A jeweils für einen C₁-C₄-Alkylenrest und R² sowie R³ und R⁴ unabhängig voneinander jeweils für Methyl, Ethyl und Isopropyl stehen
das dadurch gekennzeichnet ist, dass
a) Verbindungen der Formel (III) 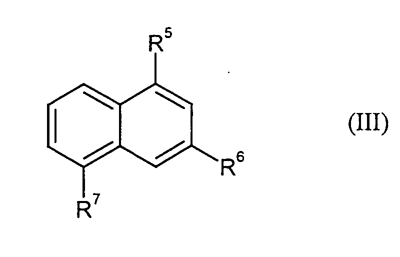 in der
   - R⁵, R⁶ und R⁷: jeweils unabhängig voneinander für Wasserstoff, Hydroxy, Amino oder SO₃M steht, wobei M für Wasserstoff, Ammonium, ein Alkalimetall oder ein halbes Äquivalent eines Erdalkalimetalls stehen kann,
   mit Alkalimetallhydroxid und gegebenenfalls Erdalkalimetallhydroxid in Gegenwart von Wasser umgesetzt werden und
b) die in Schritt a) erhaltenen Reaktionsmischungen,
   - gegebenenfalls nach Zugabe von Wasser und
   - gegebenenfalls nach der Abtrennung von unlöslichen Bestandteilen und
   - gegebenenfalls nach der Abtrennung von unerwünschten, löslichen Bestandteilen
   teilweise neutralisiert werden und
c) die in Schritt b) erhaltenen Reaktionsmischungen mit Verbindungen der Formeln (IVa), (IVb) oder (IVc)

   R¹-X (IVa)

   R¹-OSO₂-R⁸ (IVb)

   R¹-OSO₂-OR¹ (IVc),

   in denen R¹ die oben genannte Bedeutung besitzt und
   - X: für Chlor, Brom oder Iod und
   - R⁸: für C₁-C₄-Alkyl, C₁-C₄-Perfluoralkyl, Phenyl oder p-Tosyl steht,
   umgesetzt werden und
d) die in Schritt c) erhaltenen Reaktionsmischungen angesäuert werden.

**Alkyl** bzw. Alkylen bedeutet im Rahmen der Erfindung jeweils unabhängig einen geradkettigen, cyclischen, verzweigten oder unverzweigten Alkyl- bzw. Alkylen-Rest. Gleiches gilt für den Alkylenteil eines Arylalkyl-Restes.

Beispielsweise steht in allen Zusammenhängen bevorzugt C₁-C₁₄-Alkyl für Methyl, Ethyl, n-Propyl, Isopropyl, n-Butyl, tert.-Butyl, n-Pentyl, Cyclohexyl, n-Hexyl, n-Heptyl, n-Octyl, iso-Octyl, n-Decyl und n-Dodecyl.

Beispielsweise steht C₁-C₄-Alkylen in allen Zusammenhängen bevorzugt für Methylen, 1,1-Ethylen, 1,2-Ethylen, 1,1-Propylen, 1,2-Propylen, 1,3-Propylen, 1,1-Butylen, 1,2-Butylen, 2,3-Butylen und 1,4-Butylen.

**Arylalkyl** steht im Rahmen der Erfindung beispielsweise und bevorzugt für Alkylreste, die durch carbocyclische aromatische Reste mit 6 bis 10 Kohlenstoffatomen wie insbesondere Phenyl oder Naphthyl gemäß obiger Definition substituiert sind, wobei die carbocyclischen aromatischen Reste ihrerseits mit bis zu fünf Substituenten pro Cyclus substituiert sein können, die ausgewählt sind aus der Gruppe Methyl, Ethyl, Fluor, Chlor, Brom und C₁-C₄-Fluoralkyl, wobei Fluoralkyl für einen einfach mehrfach oder vollständig durch Fluor substituierten Alkylrest gemäß obiger Definition steht. Bevorzugte C₁-C₄-Fluoralkylreste sind Trifluormethyl, Pentafluorethyl, 2,2,2-Trifluorethyl und Nonafluorbutyl. Gleiches gilt für den Arylteil eines Diarylalkyl-Restes.

Besonders bevorzugt werden für Schritt a) 3-Amino-1,5-naphtalindisulfonsäure und deren Mono- oder Dialkalimetallsalze, sowie 1,3,5-Naphtalintrisulfonsäure und deren Mono-, Di- oder Tri-Alkalimetallsalze eingesetzt. Auch der Einsatz von Mischungen dieser Verbindungen ist möglich.

Ganz besonders bevorzugt werden die Trialkalimetallsalze der 1,3,5-Naphtalintrisulfonsäure wie beispielsweise Trinatrium-1,3,5-naphtalin-trisulfonat und Trikalium-1,3,5-naphtalin-trisulfonat eingesetzt, wobei Trinatrium-1,3,5-naphtalin-trisulfonat noch weiter bevorzugt ist.

Einige der Verbindungen der Formel (II) können in Form von Hydraten auftreten, die nichts eigens erwähnt werden, aber im Rahmen der Erfindung mitumfasst sind.

Die Verbindungen der Formel (II) sind entweder kommerziell verfügbar, oder nach Literaturvorschriften herstellbar.

In einer bevorzugten Ausführungsform des erfindungsgemäßen Verfahrens werden die Naphthalin-1,3,5-trisulfonsäure oder deren Mono-, Di- oder Tri-Alkalimetallsalze so hergestellt dass
i) Naphthalin mit rauchender Schwefelsäure zu Naphthalin-1,3,5-trisulfonsäure umgesetzt wird und
ii) die in Schritt i) erhaltene Naphthalin-1,3,5-trisulfonsäure gegebenenfalls in ein Mono-, Di- oder Tri-Alkalimetallsalz überführt wird.

An dieser Stelle sei darauf hingewiesen, dass beliebige Kombination der Merkmale und der genannten Vorzugsbereiche ebenfalls vom Rahmen der Erfindung mitumfasst sind.

Schritt i) kann vorteilhafterweise durch Umsetzung von Naphthalin mit rauchender Schwefelsäure erfolgen.

Beispielsweise kann man dabei so vorgehen, dass man rauchende Schwefelsäure vorlegt und Naphthalin zugibt oder konzentrierte Schwefelsäure und Naphthalin vorlegt und rauchende Schwefelsäure zugibt oder konzentrierte Schwefelsäure vorlegt und Naphthalin und rauchende Schwefelsäure zugibt.

Bevorzugt geht man im Schritt i) so vor, dass man konzentrierte Schwefelsäure vorlegt und Naphthalin und rauchende Schwefelsäure zugibt.

Konzentrierte Schwefelsäure bedeutet im Rahmen der Erfindung beispielsweise eine Schwefelsäure mit 90 bis 100 Gew.-% H₂SO₄. Rauchende Schwefelsäure bedeutet im Rahmen der Erfindung eine Schwefelsäure, die einen Gehalt von über 100 Gew.-% bezogen auf reine H₂SO₄ besitzt. Eine gängige Bezeichnung für rauchende Schwefelsäure im Sinne der Erfindung ist auch Oleum.

Üblicherweise wird bei handelsüblichem Oleum der Gehalt an freiem SO₃ angegeben, der beispielsweise 30 oder 65 Gew.-% beträgt.

Bevorzugt verwendet man im Schritt a) soviel Oleum, dass das molare Verhältnis von freiem SO₃ zu Naphthalin zwischen 1,5:1 und 10:1, bevorzugt zwischen 2:1 und 5:1 und besonders bevorzugt zwischen 2,5:1 und 4:1 liegt.

Die Temperatur bei der Zugabe kann beispielsweise -20 bis 70°C betragen, 20 bis 55°C sind bevorzugt.

Die Zeit für die Zugabe kann beispielsweise zwischen 10 min und 48 h liegen, bevorzugt sind 2h bis 24 Stunden.

Anschließend kann die resultierende Reaktionsmischung gegebenenfalls noch erhitzt werden. Die Temperatur kann dabei beispielsweise zwischen 55 und 150°C bevorzugt zwischen 80 und 100°C liegen.

Aus der resultierenden Reaktionsmischung kann die Naphthalin-1,3,5-trisulfonsäure zum Beispiel durch Zugabe von Wasser gewonnen werden.

Die Herstellung von Alkali- oder Erdalkalimetallsalzen der Naphthalin-1,3,5-trisulfonsäure gemäß Schritt ii) kann entweder aus der isolierten Naphthalin-1,3,5-trisulfonsäure oder direkt aus der resultierenden Reaktionsmischung aus Schritt i) erfolgen. Die Herstellung von Alkali- oder Erdalkalimetallsalzen der Naphthalin-1,3,5-trisulfonsäure aus der resultierenden Reaktionsmischung aus Schritt i) ist bevorzugt.

Schritt ii) kann beispielsweise derart erfolgen, dass die resultierende Reaktionsmischung aus Schritt i) z.B. durch Gießen in Wasser oder auf Eis verdünnt wird, und anschließend mit Alkalimetallhydroxiden, -hydrogencarbonaten oder -carbonaten oder wässrige Lösungen davon umgesetzt wird.

Bevorzugt werden Alkalimetallhydroxide wie insbesondere Natriumhydroxid und Kaliumhydroxid oder deren wässrige Lösungen eingesetzt.

Besonders bevorzugt ist die Umsetzung mit wässrigen Lösungen von Natriumhydroxid.

Der Gehalt der Lösungen an Alkalimetallhydroxiden kann beispielsweise zwischen 2 und 75 Gew.-% betragen, bevorzugt sind 25 bis 60 Gew.-%.

Die Temperatur der Umsetzung für Schritt ii) kann beispielsweise 0 bis 100°C betragen, bevorzugt sind 80 bis 100°C.

Die Menge des eingesetzten Alkalimetallhydroxids kann beispielsweise das 2 bis 10-fache bezogen auf das molare Verhältnis des in Schritt i) eingesetzten Naphthalins sein, bevorzugt das 2,8 bis 3,5-fache.

Nach in an sich bekannter Aufarbeitung, die zum Beispiel durch Filtration und gegebenenfalls Waschen und Trocknung des ausgefallenen Feststoffs erfolgen kann erhält man Alkalimetallsalze der Naphthalin-1,3,5-trisulfonsäure, die entweder gelagert oder vorzugsweise weiter umgesetzt werden können.

Gegebenenfalls können die gemäß Schritt ii) erhaltenen Alkalimetallsalze der Naphthalin-1,3,5-trisulfonsäure z.B. durch Umkristallisation noch weiter gereinigt werden, für den Einsatz in Schritt a) des erfindungsgemäßen Verfahrens ist dies jedoch nicht nötig.

Im **Schritt a)** des erfindungsgemäßen Verfahrens werden die Verbindungen der Formel (II) mit Alkalimetallhydroxid und gegebenenfalls Erdalkalimetallhydroxid in Gegenwart von Wasser umgesetzt.

Als Alkalimetallhydroxid können beispielsweise und bevorzugt Natrium- und Kaliumhydroxid oder Mischungen davon z.B. als Feststoff und in Form wässriger Lösungen eingesetzt werden.

Die Menge an Alkalimetallhydroxid für Schritt a) kann beispielsweise so gewählt werden, dass für jeden Rest R¹ in den Verbindungen der Formel (III), der für Amino oder SO₃M steht, 2 bis 30 mol, bevorzugt 3 bis 10 mol, insgesamt jedoch mindestens 2 mol Alkalimetallhydroxid pro Mol der Verbindung der Formel (II) eingesetzt werden. Steht M für Wasserstoff muss die Menge an Alkalimetallhydroxid vorteilhafterweise molar entsprechend erhöht werden.

Bevorzugt kann weiterhin auch der Zusatz von Erdalkalimetallhydroxiden erfolgen. Geeignete Erdalkalimetallhydroxide sind beispielsweise Magnesiumhydroxid und Calciumhydroxid, bevorzugt ist Calciumhydroxid.

Die Menge an Erdalkalimetallhydroxid für Schritt a) kann beispielsweise so gewählt werden, dass für jeden Rest R¹ in den Verbindungen der Formel (III), der für Amino oder SO₃M steht, 0,5 bis 20 mol, bevorzugt 1 bis 20 mol und besonders bevorzugt 1,5 bis 7 mol, insgesamt jedoch mindestens 1 mol Erdalkalimetallhydroxid pro Mol der Verbindung der Formel (II) eingesetzt werden.

Das molare Verhältnis von Wasser zur Verbindung der Formel (III) kann 0,5 bis 200 mol, bevorzugt 3 bis 50 mol betragen.

In einer besonders bevorzugten Ausführungsform beträgt das Verhältnis von Wasser zu der Summe aus Alkalimetallhydroxid und gegebenenfalls zugesetztem Erdalkalimetallhydroxid 1:1,4 bis 1 :6,0.

Der Druck bei der Reaktion kann beispielsweise 1 bis 200 bar betragen, bevorzugt 1 bis 100 bar und besonders bevorzugt derjenige Druck, der sich durch Aulheizen des Reaktionsgemisches auf die Reaktionstemperatur in einem geschlossenen Gefäß ausgehend von Umgebungsdruck einstellt.

Als geschlossenes Gefäß kommt zum Beispiel ein Autoklav in Frage, der z.B. aus Nickel, Nickel-Basislegierungen, Silber oder anderen, gegen Alkalien resistenten Material sein kann.

Die Temperatur der Umsetzung kann beispielsweise 240 bis 350°C betragen, bevorzugt 270 bis 320°C.

Die Reaktionszeit kann beispielsweise 2 bis 25 Stunden, bevorzugt 3 bis 8 Stunden betragen.

Im **Schritt b)** des erfindungsgemäßen Verfahrens werden die gemäß Schritt a) erhaltenen Reaktionsmischungen gegebenenfalls nach Zugabe von Wasser und gegebenenfalls nach der Abtrennung von unlöslichen Bestandteilen und gegebenenfalls der Abtrennung von unerwünschten, löslichen Bestandteilen mit Säure zumindest teilweise neutralisiert.

In einer bevorzugten Ausfuhrungsform wird gegebenenfalls nach Abkühlen der Reaktionsmischung Wasser zugesetzt, was z.B. durch Eingießen der Reaktionsmischung in Wasser oder auf Eis erfolgen kann.

Weiterhin bevorzugt werden unlösliche Bestandteile abgetrennt. Das kann beispielsweise und bevorzugt durch Filtration, Zentrifügation, Sedimentation und Dekantieren gegebenenfalls in Gegenwart von Hilfsmitteln erfolgen. Hilfsmittel können beispielsweise sein Kieselgur, wie zum Beispiel Celite®, Aktivkohle, wie zum Beispiel Norite® , Bleicherde, Montmorillonit oder Knochenkohle erfolgen. Bevorzugt ist die Filtration, besonders bevorzugt die Filtration in Gegenwart von Hilfsmitteln, bevorzugt Aktivkohle.

Ebenfalls bevorzugt ist die Abtrennung unerwünschter, löslicher Bestandteile. Unerwünschte, lösliche Bestandteile sind z.B. gefärbte, organische Nebenprodukte. Die Abtrennung unerwünschter, löslicher Bestandteile kann beispielsweise vor oder nach der teilweisen Neutralisation vorgenommen werden. Dazu geht man beispielsweise so vor, dass mit organischem Lösungsmittel extrahiert wird. Geeignete organische Lösungsmittel sind beispielsweise Ester wie Essigester und Butylacetat, aliphatische oder aromatische, gegebenenfalls halogenierte Kohlenwasserstoffe wie beispielsweise Benzin, Benzol, Toluol, Xylole, Chlorbenzol, Dichlorbenzole, Iso-propylbenzol, Petrolether, Hexan, Heptan, Octan, iso-Octan, Cyclohexan, Methylcyclohexan, Dichlormethan, Chloroform oder Tetrachlorkohlenstoff; Ether, wie Diethylether, Methyl-tert.-Butylether oder Diisopropylether Ketone, wie 2-Butanon oder Methyl-isobutyl-keton oder Mischungen solcher Lösungsmittel.

Weiterhin kann die Reaktionsmischung mit einem geeigneten Adsorbens z.B. von Verfärbungen befreit werden. Geeignete Adsorbentien sind beispielsweise Kieselgele, Aluminiumoxide, Cellulose oder Aktivkohle.

Die teilweise Neutralisation erfolgt bevorzugt dadurch, dass auf einen pH-Wert von 8 bis 13, bevorzugt 9,5 bis 11,5 eingestellt wird. Die pH-Wert-Angaben beziehen sich dabei auf Werte bei 25°C. Vorzugsweise werden zur teilweisen Neutralisation Säuren oder saure Salze mit einen mit einem pKa-Wert in Wasser von 5 oder weniger eingesetzt. Bevorzugt werden zur teilweisen Neutralisation Schwefelsäure, Salzsäure, Phosphorsäure, Salpetersäure und Bromwasserstoffsäure besonders bevorzugt Schwefelsäure und Salzsäure, ganz besonders bevorzugt Schwefelsäure eingesetzt.

In einer ganz besonders bevorzugten Ausführungsform wird Schritt b) so durchgeführt, dass das aus Schritt a) erhaltene Reaktionsgemisch zunächst mit Wasser verdünnt wird, unlösliche Bestandteile abgetrennt werden, ein pH-Wert von 8 bis 13 eingestellt wird und unerwünschte, lösliche Bestandteile abgetrennt werden.

Im Schritt c) erfolgt die Umsetzung des Reaktionsgemisches aus Schritt b) mit Verbindungen der Formeln (IVa), (IVb) oder (IVc).

In den Verbindungen der Formeln (IVa), (IVb) und (IVc) steht R¹ bevorzugt für Methyl, Ethyl, iso-Propyl oder Benzyl, besonders bevorzugt für Methyl.

Bevorzugte Verbindungen der Formel (IVa) sind Methylchlorid und Methyliodid, bevorzugte Verbindungen der Formel (IVb) sind Methylmesylat und Methyl-p-tosylat, die bevorzugte Verbindung der Formel (IVc) und für das erfindungsgemäße Verfahren ganz besonders bevorzugt ist Dimethylsulfat.

Die Menge an Verbindung der Formel (IVa), (IVb) oder (IVc) wird beispielsweise und bevorzugt so gewählt, dass das molare Verhältnis zur ursprünglich eingesetzten Verbindung der Formel (111)1:1 bis 10:1, bevorzugt 2:1 bis 7:1 beträgt.

Die Reaktionstemperatur in Schritt c) beträgt beispielsweise 0 bis 120°C, bevorzugt 20 bis 100°C. Bei Einsatz von bei Reaktionstemperatur gasförmigen Verbindungen der Formeln (IVa), (IVb) und (IVc) wie insbesondere Methylchlorid kann die Reaktion vorteilhafterweise unter Druck durchgeführt werden.

Ansonsten ist Umgebungsdruck bevorzugt, wenngleich der Reaktionsdruck nicht kritisch ist.

Die Reaktionszeit für Schritt c) kann beispielsweise 1 bis 25 Stunden betragen, bevorzugt sind 2 bis 10 Stunden.

Weiterhin ist bevorzugt, den pH-Wert im Laufe der Reaktion zwischen 8 und 13, bevorzugt 9,5 und 11,5 zu halten, was beispielsweise durch Zugabe von Base erfolgen kann.

Als Base eignen sich insbesondere Alkalimetallhydroxide, -carbonate oder -hydrogencarbonate, wobei Natriumhydroxid und Kaliumhydroxid bevorzugt sind. Der Einsatz der Base kann beispielsweise in fester Form oder in Form wässriger Lösungen erfolgen.

Anschließend wird im **Schritt d)** die im Schritt c) erhaltene Reaktionsmischung angesäuert.

Bevorzugt wird auf einen pH-Wert von 3,5 oder weniger, besonders bevorzugt 0 bis 3,5 und ganz besonders bevorzugt 1 bis 2,5 angesäuert.

Zum Ansäuern eignen sich beispielsweise Säuren oder saure Salze mit einem pKa-Wert von 3,5 oder weniger, bevorzugt 0 oder weniger. Besonders bevorzugt werden Schwefelsäure oder Salzsäure eingesetzt.

In einer bevorzugten Ausführungsform im Schritt d) wird zunächst nur auf einen pH-Wert von über 3,5 bis unter 8 angesäuert und unerwünschte, lösliche Bestandteile analog wie oben beschrieben extraktiv entfernt. Anschließend kann die Reaktionsmischung dann mit einem geeigneten Adsorbens z.B. von Verfärbungen befreit werden. Geeignete Adsorbentien sind beispielsweise Kieselgele, Aluminiumoxide, Cellulose oder Aktivkohle.

Anschließend wird dann weiter angesäuert.

Die Temperatur beim Ansäuern ist nicht kritisch, es kann jedoch vorteilhaft sein, die Reaktionsmischung zum Sieden zu erhitzen um gelöste Gase auszutreiben oder gegebenenfalls vorhandenes Sulfit zu zersetzen. Durch das Ansäuern werden die Salze der Verbindungen der Formel (I) protoniert und zumindest teilweise in die freien Säuren der Formel (I) überführt.

Bevorzugt werden für Schritt d) Säuren mit einem pKa-Wert in Wasser von 3 oder weniger verwendet, besonders bevorzugt Salzsäure oder Schwefelsäure, wobei Schwefelsäure noch weiter bevorzugt ist.

Aus den gemäß Schritt d) erhaltenen Reaktionsgemischen können die Verbindungen der Formel (I) in an sich bekannter Weise beispielsweise durch Extraktion mit organischem Lösungsmittel, Filtration, Zentrifügation oder Sedimentation und Dekantieren gewonnen werden.

Für die Extraktion bevorzugte Lösungsmittel sind beispielsweise Ester wie Essigester und Butylacetat, aliphatische oder aromatische, gegebenenfalls halogenierte Kohlenwasserstoffe wie beispielsweise Benzin, Benzol, Toluol, Xylole, Chlorbenzol, Dichlorbenzole, Iso-propylbenzol, Petrolether, Hexan, Heptan, Octan, iso-Octan, Cyclohexan, Methylcyclohexan, Dichlormethan, Chloroform oder Tetrachlorkohlenstoff; Ether, wie Diethylether, Methyl-tert.-Butylether oder Diisopropylether Ketone, wie 2-Butanon oder Methyl-isobutyl-keton oder Mischungen solcher Lösungsmittel.

Die Verbindungen der Formel (I) können nach Extraktion in an sich bekannter Weise beispielsweise durch Verdampfen des Lösungsmittels gewonnen werden.

Trennt man die Verbindungen der Formel (I) durch Filtration, Zentrifugation oder Sedimentation und Dekantieren ab, so kann dies beispielsweise und bevorzugt bei 0 bis 70°C, besonders bevorzugt 20 bis 55°C geschehen.

Zur weiteren Reinigung können die Verbindungen der Formel (I) gegebenenfalls umkristallisiert oder umgefällt werden, nötig ist dies jedoch nicht.

Die nach den erfindungsgemäßen Verfahren hergestellten Verbindungen der Formel (I) wie insbesondere 3-Methoxy-2-methylbenzoesäure eignen sich besonders zur Verwendung in einem Verfahren zur Herstellung von Arzneimitteln und Agrochemikalien, wie zum Beispiel Pflanzenschutzmitteln und Insektiziden oder Zwischenprodukten davon, insbesondere Säurechloride, Säurebromide, Säurehydrazide, Ester, wie z.B. 3-Methoxy-2-methyl-benzoylchlorid oder Bromid, 3-Methoxy-2-methylbenzoesäurehydrazide, 3-Methoxy-2-methyl-benzosäuremethylester.

Der Vorteil der erfindungsgemäßen Verfahren ist die effiziente Herstellung von Verbindungen der Formel (I), die die Durchführung ohne aufwändige Zwischenisolierungen in hohen Ausbeuten ermöglicht.

### Beispiele

### Beispiel 1

In einem Nickelautoklaven werden in ein Gemisch aus 232,0 g wässriger Natronlauge (45 %ig) und 194,0 g Natriumhydroxid 300,0 g Trinatrium-1,3,5-naphthalintrisulfonat (70,9 %ig, berechnet als freie Säure) so eingerührt, dass eine gut rührbare, breiartige Suspension entsteht. Der Autoklav wird verschlossen und ohne Rühren auf 190°C aufgeheizt und bei dieser Temperatur 30 Minuten temperiert. Danach wird der Rührer eingeschaltet und auf 280°C aufgeheizt. Der Innendruck steigt dabei auf 18,4 bar. Es wird 6 Stunden bei 280°C nachgerührt und danach auf 90°C abgekühlt. Bei dieser Temperatur werden 200,0 g Wasser eingepumpt und anschließend auf Raumtemperatur abgekühlt. Die so erhaltene Reaktionsmischung wird von Unlöslichem abgesaugt und mit 187,3 g Wasser nachgewaschen. Man erhält 251,8 g eines bräunlich-weißen, feinteiligen Feststoffs und 795,7 g einer dunkelbraunen Lösung.

### Beispiel 2

In einem Kolben werden 390,0 g der Lösung aus Beispiel 1 vorgelegt, mit 88,4 g Schwefelsäure (100 %ig) ein pH-Wert von 10,3 eingestellt und die Reaktionsmischung auf 40 bis 45°C erwärmt. Dann werden bei gleicher Temperatur Dimethylsulfat und Natronlauge simultan so eindosiert, dass der pH-Wert im Bereich von 10,4 bis 10,6 bleibt. Es werden in 2 Stunden insgesamt 147,5 g Dimethylsulfat und 85,8 g Natronlauge (30 %ig) zudosiert. Danach wird 1 Stunde bei der gleichen Temperatur nachgerührt. Nach beendeter Nachrührzeit wird der pH-Wert durch Zudosieren von Natronlauge auf 11 erhöht und auf 90°C aufgeheizt. Bei dieser Temperatur wird 2 Stunden nachgerührt, wobei Nachdosieren von Natronlauge (30 %ig) notwendig wird. Die Reaktionslösung wird auf 70°C abgekühlt. Man gibt 20,0 g Aktivkohle Acticarbon F© zu und stellt durch Zudosieren von 32,9 g Schwefelsäure (100 %ig) einen pH-Wert von 6,5 ein. Man erhitzt zum Rückfluss, klärt die Reaktionslösung und wäscht mit 550,0 g Wasser nach. Die geklärte Reaktionslösung wird in den Reaktor zurückgegeben auf 90°C aufgeheizt und durch Zudosieren von 18,9 g Schwefelsäure (100 %ig) ein pH von 3 eingestellt. Man tempert 1 Stunde bei 90°C. Dann wird mit 41,6 g Schwefelsäure (100 %ig) ein pH von 3 eingestellt. Man tempert 1 Stunde bei 90°C. Dann wird mit 41,6 g Schwefelsäure (100 %ig) ein pH von 1 eingestellt und die Reaktionsmischung innerhalb von 4 Stunden auf 45°C abgekühlt. Dabei fällt das Produkt als weißer Niederschlag aus. Das Produkt wird abgesaugt und mit 300,0 g Wasser nachgewaschen. Der Niederschlag wird im Vakuumtrockenschrank getrocknet. Man erhält 1532,9 g Wasch-/Mutterlauge (0,03 % Methoxymethylbenzoesäure) und 29,3 g 3-Methoxy-2-methylbenzoesäure (Gehalt 95,9 %). Das entspricht einer isolierten Ausbeute bezogen auf Trinatrium-1,3,5-naphthalintrisulfonat von 60 % d.Th.

### Beispiel 3

In einem Nickelautoklaven wird in einem Gemisch aus 356,0 g wässriger Natronlauge (45 %ig); 126,0 g Natriumhydroxid-Pastillen und 127,0 g Calciumhydroxid 300,0 g Trinatrium-1,3,5-naphthalintrisulfonat (70,9 %ig, berechnet als freie Säure) so eingerührt, dass eine gut rührbare, breiartige Suspension entsteht. Der Autoklav wird verschlossen, ohne Rühren auf 190°C aufgeheizt und bei dieser Temperatur 30 Minuten temperiert. Danach wird der Rührer eingeschaltet und auf 280°C aufgeheizt. Der Innendruck (Eigendruck) steigt dabei auf 29,6 bar. Es wird 15 Stunden bei der Reaktionstemperatur nachgerührt und danach auf 90°C abgekühlt. Bei dieser Temperatur werden 200,0 g Wasser eingepumpt und danach auf Raumtemperatur abgekühlt. Die erhaltene Suspension wird abgesaugt und mit 695,5 g Wasser nachgewaschen. Man erhält 299,6 g eines bräunlich-weißen, feinteiligen Feststoffs und 1287,5 g einer dunkelbraunen Lösung.

### Beispiel 4

In einem Kolben werden 300,0 g der Lösung aus Beispiel 3 vorgelegt und mit 50,0 g Wasser verdünnt. Mit 42,8 g Schwefelsäure (100 %ig) wird ein pH-Wert von 10,3 eingestellt und die Reaktionsmischung auf 40 bis 45°C erwärmt. Dann werden bei 40 bis 45°C Dimethylsulfat und Natronlauge simultan so eindosiert, dass der pH-Wert im Bereich von 10,4 bis 10,6 bleibt. Es werden in 2 Stunden 65,1 g Dimethylsulfat und 45,0 g Natronlauge (30 %ig) zudosiert. Danach wird 1 Stunde bei der gleichen Temperatur nachgerührt. Nach beendeter Nachrührzeit wird der pH-Wert durch Zudosieren von Natronlauge auf 11 erhöht und auf 90°C aufgeheizt. Bei dieser Temperatur wird 2 Stunden nachgerührt, wobei Nachdosieren von Natronlauge (30 %ig) benötigt wird. Die Reaktionslösung wird auf 70°C abgekühlt. Man gibt 3,0 g Aktivkohle zu und stellt durch Zudosieren von 13,1 g Schwefelsäure (100 %ig) einen pH-Wert von 7,8 ein. Man erhitzt zum Rückfluss, klärt die Reaktionslösung mit Aktivkohle und wäscht mit 250 g Wasser nach. Die geklärte Reaktionslösung wird in den Reaktor zurückgegeben, auf 90°C aufgeheizt und durch Zudosieren von 11,4 g Schwefelsäure (100 %ig) ein pH von 4 eingestellt. Man tempert 1 Stunde bei 90°C. Dann wird mit 38,4 g Schwefelsäure (100 %ig) ein pH-Wert von 1 eingestellt und die Reaktionsmischung innerhalb von 4 Stunden auf 45°C abgekühlt. Dabei fällt das Produkt als weißer Niederschlag aus. Das Produkt wird auf einer Glasfilternutsche abgesaugt und mit 250 g Wasser nachgewaschen. Der Niederschlag wird im Vakuumtrockenschrank getrocknet. Man erhält 784,7 g Mutterlauge (0,02 % Methoxymethylbenzoesäure), 238,0 g Waschlauge (Methoxymethylbenzoesäure nicht nachweisbar) und 16,9 g 3-Methoxy-2-methylbenzoesäure (Gehalt 95,5 %). Das entspricht einer isolierten Ausbeute bezogen auf Trinatrium-1,3,5-naphthalintrisulfonat von 72,1 % d.Th.

### Beispiel 5 (3-Methoxy-2-methylbenzoemethylester)

In einem Planschliffbecher werden in einem Gemisch aus 200,0 g Wasser und 173,3 g Natronlauge (w = 30 %) 200,0 g 3-Hydroxy-2-methylbenzoesäure (Gehalt 96,9 %) aufgelöst:

Dabei stellt sich ein pH von ca. 5,5 ein. Die klare Lösung wird auf 40°C erwärmt und durch Zudosieren von Natronlauge (w = 30 %) auf pH = 10,5 eingestellt. Jetzt werden bei 40-45°C Dimethylsulfat und Natronlauge simultan so eindosiert, dass der pH-Wert im Bereich von 10,4-10,6 bleibt. Es werden in 2 h 401,5 g Dimethylsulfat und 147,9 g Natronlauge (w = 30 %) zudosiert. Danach wird bei 40-45°C 2 h nachgerührt. Danach wird die wässrige Phase abgetrennt und verworfen. Die organische Phase wird mit 200,0 g Wasser gewaschen. Das Waschwasser wird abgetrennt und verworfen. Es bleiben 223,56 g roher 3-Methoxy-2-methylbenzoesäuremethylester zurück.

Zur weiteren Aufreinigung wird das Rohprodukt einer Vakuumdestillation über eine Vigreux-Kolonne unterworfen. Bei einem Vakuum von 20 mbar gehen 5,7 g Vorlauf und 205,5 g Hauptlauf (145°C Kopftemperatur, 3-Methoxy-2-methylbenzoesäuremethylester, Gehalt 98,7 %) über.

205,0 g 3-Methoxy-2-methylbenzoesäuremethylester, Gehalt 98,7 % MMBE und 0,00 % 3-Hydroxy-2-methylbenzoesäure, 3-Hydroxy-2-methylbenzoesäuremethylester, 3-Methoxy-2-methylbenzoesäure, entspricht 88,5 % d.Th. (bez. auf 3-Hydroxy-2-methylbenzoesäure).

### Beispiel 6 (3-Methoxy-2-methylbenzoesäure)

In einem Planschliffbecher werden in einem Gemisch aus 200,0 g Wasser und 173,3 g Natronlauge (w = 30 %) 213,1 g 3-Hydroxy-2-methylbenzoesäure (Gehalt 92,75 %) aufgelöst:

Dabei stellt sich ein pH von ca. 5,5 ein. Die klare, braune Lösung wird auf 40°C erwärmt und durch Zudosieren von Natronlauge (w = 30 %) auf pH = 10,5 eingestellt. Jetzt werden bei 40-45°C Dimethylsulfat und Natronlauge simultan so eindosiert, dass der pH-Wert im Bereich von 10,4-10,6 bleibt. Es werden in 2 h 401,5 g Dimethylsulfat und 127,2 g Natronlauge (w = 30 %) zudosiert. Danach wird bei 40-45°C 2 h nachgerührt. Danach wird die wässrige Phase abgetrennt und verworfen. Zu der organischen Phase gibt man 250,0 g Wasser und heizt auf 90°C auf. Man rührt das Gemisch bei dieser Temperatur 2 h nach und hält den pH-Wert dabei durch Zudosieren von Natronlauge (w = 30%) bei 10,5-11. Dabei verschwindet die organische Phase und es bildet sich eine Lösung. Die Reaktionslösung wird auf 80°C abgekühlt.

In einem zweiten Planschliffbecher werden 250,0 g Wasser vorgelegt und auf 80°C aufgeheizt. Hierzu dosiert man simultan das Reaktionsgemisch des ersten Bechers und Schwefelsäure (w = 100 %) bei 80°C so ein, dass ein pH von 3,8-4,2 eingehalten wird. Dabei fällt das Produkt als weißer Niederschlag aus. Nach Eindosieren von ca. 10 % der Reaktionslösung wird ca. 1 h getempert. Nach vollständiger Dosierung wird mit Schwefelsäure (w = 100 %) der pH auf 1 eingestellt und innerhalb von 4 h auf 40°C abgekühlt. Das Produkt wird abgesaugt und mit 2 x 500,0 g Wasser nachgewaschen. Der Niederschlag wird im Vakuumtrockenschrank 16 h bei 60°C getrocknet. Man erhält 202,0 g 3-Methoxy-2-methylbenzoesäure, Gehalt 96,6 % MMBS (0,00 % 3-Hydroxy-2-methylbenzoesäure, 3-Hydroxy-2-methylbenzoesäuremethylester, 3-Methoxy-2-methylbenzoesäuremethylester) entspricht 90,4 % d.Th. (bez. auf 3-Hydroxy-2-methylbenzoesäure).

### Beispiel 7 (3 -Methoxy-2-methylbenzoylchlorid)

In einem Dreihalskolben werden unter Stickstoff 136,0 g Thionylchlorid vorgelegt und auf 60°C aufgeheizt. Dazu tropft man eine 180°C heiße Schmelze von 136,0 g 3-Methoxy-2-methylbenzoesäure in einer Stunde zu. Die Reaktion setzt unter Gasentwicklung (SO₂, HCl) sofort ein. Nach Beendigung der Zugabe wird innerhalb einer Stunde zum Rückfluss (80°C) erhitzt und bei dieser Temperatur bis zum Abklingen der Gasentwicklung (ca. 2 h) gerührt. Die Lösung wird unter Stickstoff auf ca. 50°C abgekühlt und der Rückflusskühler durch eine Vigreux-Kolonne ersetzt. Es werden bei Normaldruck (Sumpf bis 170°C) 14,0 g Thionylchlorid abdestilliert und der zurückbleibende Sumpf im Vakuum (10 mbar) fraktioniert. Man erhält als Destillat 139,4 g 3-Methoxy-2-methylbenzoylchlorid (98,8 %ig). Das entspricht 94,2 % d. Th.

### Beispiel 8 (3 -Methoxy-2-methylbenzoylchlorid)

In einem Kolben werden unter Stickstoff in 200,0 g trockenem Xylol 91,9 g 3-Methoxy-2-methylbenzoesäure suspendiert. Dazu dosiert man bei Raumtemperatur 119,0 g Thionylchlorid. Die graue Suspension wird innerhalb einer Stunde auf 80°C aufgeheizt, wobei die Reaktion unter Gasentwicklung (SO₂, HCl) bei ca. 30°C einsetzt und bei etwa 50°C eine homogene Lösung entsteht. Danach wird in einer weiteren Stunde die Reaktionstemperatur bis auf 135°C angehoben. Bei dieser Temperatur wird bis Beendigung der weitergerührt (1 h). Danach wird der Rückflusskühler durch eine kleine Vigreux-Kolonne mit Destillationsbrücke ersetzt und zuerst die überschüssigen ca. 45,0 g Thionylchlorid und danach bei einer Sumpftemperatur von maximal 154°C der größte Teil des Xylols (ca. 165 g) abdestilliert. Die zurückbleibenden 180,0 g Sumpf bestehen bei angenommenem, quantitativem Umsatz zu 54,4 % aus 3-Methoxy-2-methylbenzoylchlorid. Das entspricht 0,53 mol, bzw. 97,8 g 3-Methoxy-2-methylbenzoylchlorid 100 %ig. Der Sumpf ist bei Temperaturen von ca. 30°C flüssig und wird für die weitere Reaktion direkt eingesetzt.

### Beispiel 9 (3-Methoxy-2-methylbenzoesäure-tert.-butylhydrazid)

In einem Planschliffbecher werden in 230,0 g Wasser 81,3 g tert.-Butylhydrazin-Hydrochlorid gelöst. Durch Zudosieren von 55,8 g Natronlauge (w = 30 %) wird ein pH-Wert von 9,2 eingestellt. Man gibt zur Lösung 150,0 g Spezialbenzin 80/110 (Siedebereich 80-110°C) zu und kühlt das Gemisch auf 15°C ab. Jetzt werden bei 15-20°C die unter Beispiel 1 hergestellte xylolische 3-Methoxy-2-methylbenzoylchlorid-Lösung und Natronlauge simultan so eindosiert, dass der pH-Wert im Bereich von 9,0-9,5 bleibt. Es werden in 1,5 h 180,0 g xylolische MMBC-Lösung (w = 30 %) und 97,3 g Natronlauge (w = 30 %) zudosiert. Die Pumpe und die Zuleitungen werden mit 43,0 g Xylol gespült, dieses Xylol wird zur Reaktionsmischung zugegeben. Nach Dosierende wird noch eine Stunde bei 10-15°C nachgerührt.

Danach wird die Reaktionsmischung in 45 min auf 40°C erwärmt und bei dieser Temperatur 2 h zur Vervollständigung der Reaktion gerührt.

Die Suspension wird auf 70°C aufgeheizt, wobei sich der Feststoff vollständig in der organischen Phase löst. Die Wasserphase wird abgetrennt und verworfen. Die organische Phase wird mit 200,0 g VE-Wasser gewaschen, das Waschwasser wird ebenfalls verworfen.

Zur organischen Phase werden 2,0 g Aktivkohle zugegeben, die Mischung auf 80°C aufgeheizt, und über ein Papierfilter filtriert. Der Filterkuchen wird mit 43,0 g Xylol nachgewaschen, die Filtrate werden vereinigt und weiterverarbeitet.

Zur geklärten organische Phase gibt man bei 60-80°C 300,0 g Spezialbenzin 80/110 und kühlt innerhalb von 6 h auf 5°C ab. Bei ca. 38-40°C beginnt das Produkt auszufallen. Das ausgefällte Produkt wird abgesaugt und mit Spezialbenzin 80/110 nachgewaschen. Der Niederschlag wird im Vakuumtrockenschrank bei 200 mbar 20 h bei 60°C getrocknet. Man erhält 109,2 g 3-Methoxy-2-methylbenzoesäure-tert.butylhydrazid (Gehalt 95,4 %, im HPLC eine weitere Komponente erkennbar). Das entspricht einer Ausbeute bezogen auf 3-Methoxy-2-methylbenzoesäure von 83,2 % d.Th.

## Patentansprüche

1. Verfahren zur Herstellung von Verbindungen der Formel (I), 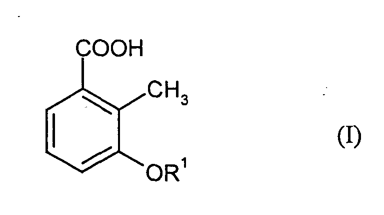 in der
R¹ für C₁-C₁₄-Alkyl, C₇-C₂₀-Arylalkyl, C₁₃-C₂₀-Diarylalkyl oder Reste der Formeln (IIa) oder (IIb) steht
A-OR² (IIa)
A-NR³R⁴ (IIb)
in der
A jeweils für einen C₁-C₄-Alkylenrest und R² sowie R³ und R⁴ unabhängig voneinander jeweils für Methyl, Ethyl und Isopropyl stehen
**dadurch gekennzeichnet, dass**
a) Verbindungen der Formel (III) 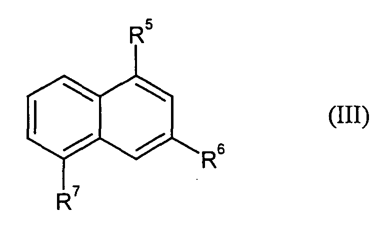 in der
R⁵, R⁶ und R⁷ jeweils unabhängig voneinander für Wasserstoff, Hydroxy, Amino oder SO₃M stehen, wobei M für Wasserstoff, Ammonium, ein Alkalimetall oder ein halbes Äquivalent eines Erdalkalimetalls steht,
mit Alkalimetallhydroxid in Gegenwart von Wasser umgesetzt werden und
b) die in Schritt a)
c) die in Schritt b) erhaltenen Reaktionsmischungen mit Verbindungen der Formeln (IVa), (IVb) oder (IVc)
R¹-X (IVa)
R¹-OSO₂-R⁸ (IVb)
R¹-OSO₂-OR¹ (IVc),
in denen
R¹ die oben genannte Bedeutung besitzt und
X für Chlor, Brom oder Iod und
R⁸ für C₁-C₄-Alkyl, C₁-C₄-Perfluoralkyl, Phenyl oder p-Tosyl steht,
umgesetzt werden und
d) die in Schritt c) erhaltenen Reaktionsmischungen angesäuert werden.

2. Verfahren gemäß Anspruch 1, **dadurch gekennzeichnet, dass** Schritt a) in Gegenwart von Erdalkalimetallhydroxid durchgeführt wird.

3. Verfahren gemäß einem oder mehreren der Ansprüche 1 bis 2, **dadurch gekennzeichnet, dass** Schritt a) in Gegenwart von Caiciumhydroxid durchgeführt wird.

4. Verfahren gemäß einem oder mehreren der Ansprüche 1 bis 3, **dadurch gekennzeichnet, dass** im Schritt a) 3-Amino-1,5-naphtalindisulfonsäure, deren Mono- oder Dialkalimetallsalze, 1,3,5-Naphtalintrisulfonsäure öder deren Mono-, Di- oder Tri-Alkalimetallsalze eingesetzt werden.

5. Verfahren gemäß einem oder mehreren der Ansprüche 1 bis 4, **dadurch gekennzeichnet, dass** im Schritt a) Trialkalimetallsalze der 1,3,5-Naphthalintrisulfonsäure eingesetzt werden.

6. Verfahren gemäß einem oder mehreren der Ansprüche 1 bis 5, **dadurch gekennzeichnet, dass** im Schritt a) als Alkalimetallhydroxid Natrium- oder Kaliumhydroxid oder Mischungen davon eingesetzt werden.

7. Verfahren gemäß einem oder mehreren der Ansprüche 1 bis 6, **dadurch gekennzeichnet, dass** im Schritt b) unlösliche Bestandteile abgetrennt werden.

8. Verfahren gemäß einem oder mehreren der Ansprüche 1 bis 7, **dadurch gekennzeichnet, dass** im Schritt b) ein pH-Wert von 8 bis 13 eingestellt wird.

9. Verfahren gemäß einem oder mehreren der Ansprüche 1 bis 8, **dadurch gekennzeichnet, dass** im Schritt c) Methylchlorid, Methyliodid, Methylmesylat, Methyl-p-tosylat oder Dimethylsulfat eingesetzt werden.

10. Verfahren gemäß einem oder mehreren der Ansprüche 1 bis 9, **dadurch gekennzeichnet, dass** im Schritt c) der pH-Wert im Laufe der Reaktion zwischen 8 und 13 gehalten wird.

11. Verfahren gemäß einem oder mehreren der Ansprüche 1 bis 10, **dadurch gekennzeichnet, dass** im Schritt d) zunächst auf einen pH-Wert von über 3,5 bis unter 8 angesäuert wird und unerwünschte, lösliche Bestandteile extraktiv entfernt werden.

12. Verwendung von Verbindungen der Formel (I), mit der unter Anspruch 1 genannten Bedeutung, die nach einem Verfahren gemäß einem oder mehreren der Ansprüche 1 bis 11 hergestellt wurden, in einem Verfahren zur Herstellung von Arzneimitteln und Agrochemikalien.
